# EUROPEAN PATENT SPECIFICATION

(11) **EP 1 487 809 B1**
(45) Date of publication and mention of the grant of the patent: **09.01.2008**
(21) Application number: 03745297.6
(22) Date of filing: 27.03.2003
(51) Int. Cl.: C07D 265/30

(54) **PROCESS FOR THE PREPARATION OF MORPHOLINE DERIVATIVES AND INTERMEDIATES THEREFORE**
VERFAHREN ZUR HERSTELLUNG VON MORPHOLINDERIVATEN UND ZWISCHENPRODUKTE DAFÜR
PROCEDE DE PREPARATION DE DERIVES DE MORPHOLINE ET PRODUITS INTERMEDIAIRES OBTENUS A CET EFFET

(30) Priority: 28.03.2002 GB 0207450
(43) Date of publication of application: 22.12.2004
(73) Proprietor: GLAXO GROUP LIMITED, Greenford, Middlesex UB6 0NN (GB)
(72) Inventor: HAYES, Martin Alistair, Stevenage, Hertfordshire SG1 2NY (GB); MILLS, Gail, Stevenage, Hertfordshire SG1 2NY (GB); SWANSON, Stephen, Stevenage, Hertfordshire SG1 2NY (GB); WALKER, Andrew John, Stevenage, Hertfordshire SG1 2NY (GB); WILKINSON, Mark, Stevenage, Hertfordshire SG1 2NY (GB)
(74) Representative: Povey, Alexander W.G.
(86) International application number: PCT/EP2003/003343
(87) International publication number: WO 2003/082835

(56) References cited:
- EP-A- 0 995 746
- WO-A-02/26723
- DE-A- 2 447 732
- KATO S ET AL: "NOVEL BENZAMIDES AS SELECTIVE AND POTENT GASTRIC PROKINETIC AGENTS 1. SYNTHESIS AND STRUCTURE-ACTIVITY RELATIONSHIPS OF N-(2-MORPHOLINYL)ALKYLBENZAMIDES" JOURNAL OF MEDICINAL CHEMISTRY, AMERICAN CHEMICAL SOCIETY. WASHINGTON, US, vol. 33, no. 5, May 1990 (1990-05), pages 1406-1413, XP001037844 ISSN: 0022-2623
- MORIE T ET AL: "ASYMMETRIC SYNTHESIS OF THE ENANTIOMERS OF 2-AMINOMETHYL-4-(4-FLUOROB ENZYL)MORPHOLINE, AN INTERMEDIATE OF MOSAPRIDE, A GASTROPROKINETIC AGENT" HETEROCYCLES, ELSEVIER SCIENCE PUBLISHERS B.V. AMSTERDAM, NL, vol. 38, no. 5, 1994, pages 1033-1040, XP001037848 ISSN: 0385-5414
- MORIE T ET AL: "Synthesis and Biological Activities of the Optical Isomers OF (PLUS MINUS)-4-AMINO-5-CHLORO-2-ETHOXY-N-ÄÄ4-(4- FLUOROBENZYL)-2-MORPHOLINYL ÜMETHYLÜBENZAMIDE (MOSAPRIDE)" CHEMICAL AND PHARMACEUTICAL BULLETIN, PHARMACEUTICAL SOCIETY OF JAPAN. TOKYO, JP, vol. 42, no. 4, 1994, pages 877-882, XP002216378 ISSN: 0009-2363
- SAKURAI N ET AL: "Synthesis and structure-activity relationships of 7-(2-aminoalkyl)morpholinoquinolones as anti-helicobacter pylori agents" BIOORGANIC & MEDICINAL CHEMISTRY LETTERS, OXFORD, GB, vol. 8, no. 16, 18 August 1998 (1998-08-18), pages 2185-2190, XP004137243 ISSN: 0960-894X

## Description

This invention relates to novel processes, In particular to processes for preparing certain morpholine derivatives.

Co-pending International Patent Application number PCT/GB01/04350 (WO 02/026725) (Glaxo Group Limited) relates to certain morpholine urea derivatives of formula (I) 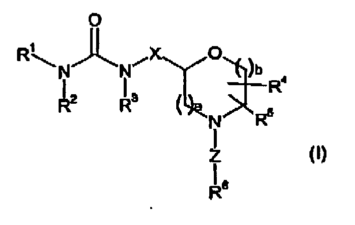 wherein:
R¹ represents C₁₋₆ alkyl, C₂₋₆ alkenyl, C₂₋₆ alkynyl, aryl-Y¹-, heteroaryl-Y¹-, aryl-(O)ₜ-aryl-Y¹-, aryl-(O)ₜ-heteroaryl-Y¹-, heteroaryl-(O)ₜ-aryl-Y¹-, heteroaryl-(O)ₜ-heteroaryl-Y¹-, aryl- SO₂-Y¹-, C₁₋₆ alkyl-G-Y¹-, heteroaryl-G-aryl-Y¹-, J¹-SO₂-Y¹-, R¹⁷O(CO)-C₂₋₆ alkenyl-Y¹-, R¹⁷NHCO-Y¹-, R¹⁷NHSO₂-Y¹-, C₂₋₆ alkynyl-Y¹-, C₂₋₆ alkenyl-Y¹-, aryl-O-Y¹-, heteroaryl-O-Y¹-, C₁₋₆ alkyl-SO₂-Y¹-, M-Y¹-, J¹-Y¹-, J¹-COY¹-, aryl-CO-Y¹- or C₃₋₈ cycloalkyl-Y¹- or C₃₋₈ cycloalkenyl-Y¹-, which C₂₋₆ alkynyl and C₂₋₆ alkynyl-Y¹ may be optionally substituted with a -OR¹⁷ group, which C₂₋₆ alkenyl may be optionally substituted by one or more -COOR¹⁷ groups and which cycloalkyl or cycloalkenyl may be optionally substituted by one or more hydroxyl or C₁₋₆ alkyl groups,
R² represents hydrogen or C₁₋₆ alkyl optionally substituted by a hydroxy group;
R³ represents hydrogen or C₁₋₆ alkyl;
or R¹ and R² may together with the nitrogen atom to which they are attached form a group of formula J² wherein said nitrogen atom substitutes for either X¹ or X²;
t represents 0 or 1;
X represents ethylene or a group of formula CR^{e}R^{f} wherein R^{e} and R^{f} Independently represent hydrogen or C₁₋₄ alkyl or R^{e} and R^{f} may together with the carbon atom to which they are attached form a C₃₋₈ cycloalkyl group; R⁴ and R⁵ independently represent hydrogen or C₁₋₄ alkyl;
Z represents a bond, CO, SO₂, CR¹⁰R⁷(CH₂)ₙ, (CH₂)ₙCR¹⁰R⁷, CHR⁷(CH₂)ₙO, CHR⁷(CH₂)ₙS, CHR⁷(CH₂)ₙOCO, CHR⁷(CH₂)ₙCO, COCHR⁷(CH₂)ₙ or SO₂CHR⁷(CH₂)ₙ;
R⁶ represents C₁₋₆ alkyl, C₂₋₆ alkenyl, aryl, heteroaryl, aryl-C₂₋₆ alkenyl-, - CN or a group of formula Y²-J³;
R⁷ represents hydrogen, C₁₋₄ alkyl, CONR⁸R⁹ or COOC₁₋₆ alkyl;
a and b represent 1 or 2, such that a+b represents 2 or 3;
G represents -SO₂-. -SO₂NR¹⁸-, -NR¹⁸SO₂-, -NR¹⁸CO-, CO or-CONR¹⁸-;
n represents an Integer from 0 to 4;
M represents a C₃₋₆ cycloalkyl or C₃₋₆ cycloalkenyl group fused to a monocyclic aryl or monocyclic heteroaryl group;
J¹, J² and J³ independently represent a moiety of formula (K): 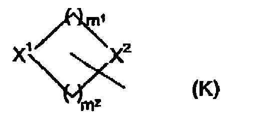 wherein X¹ represents oxygen, NR¹¹ or sulphur, X² represents CH₂, oxygen, NR¹² or sulphur, m¹ represents an integer from 1 to 3 and m² represents an integer from 1 to 3, provided that m¹+m² is in the range from 3 to 5, also provided that when both X¹ and X² represent oxygen, NR¹¹, NR¹² or sulphur, m¹ and m² must both not equal less than 2, wherein K is optionally substituted by one or more (eg. 1 or 2) -Y³-aryl, -Y³-heteroaryl, -Y³-CO-aryl, -COC₃₋₈ cycloalkyl, -Y³-CO-heteroaryl, -C₁₋₆ alkyl, -Y³-COOC₁₋₆ alkyl, -Y³-COC₁₋₆ alkyl, -Y³-W, -Y³-CO-W, -Y³-NR¹⁵R¹⁶, -Y³-CONR¹⁵R¹⁶, hydroxy, oxo, -Y³-SO₂NR¹⁵R¹⁶, Y³-SO₂C₁₋₈ alkyl, -Y³-SO₂aryl, -Y³-SO₂heteroaryl, -Y³-NR¹³C₁₋₈ alkyl, -Y³-NR¹³SO₂C₁₋₆ alkyl,-Y³-NR¹³-CONR¹⁵R¹⁶, -Y³-NR¹³COOR¹⁴ or -Y³-OCONR¹⁵R¹⁶ groups, and is optionally fused to a monocyclic aryl or heteroaryl ring; R⁸, R⁹, R¹⁰, R¹¹, R¹², R¹³ and R¹⁴ Independently represent hydrogen or C₁₋₆ alkyl;
R¹⁵ and R¹⁶ independently represent hydrogen or C₁₋₆ alkyl or R¹⁵ and R¹⁶ together with the nitrogen atom to which they are attached may form a morpholine, piperidine or pyrrolidine ring;
R¹⁷ and R¹⁸ independently represent hydrogen or C₁₋₆ alkyl;
W represents a saturated or unsaturated, non-aromatic 5-7 membered ring containing between 1 and 3 heteroatoms selected from nitrogen, oxygen or sulphur, optionally substituted with one or more C₁₋₆ alkyl, halogen or hydroxy groups;
Y¹, Y² and Y³ independently represent a bond or a group of formula - (CH₂)ₚCR^{c}R^{d}(CH₂)_{q}- wherein R^{c} and R^{d} independently represent hydrogen or C₁₋₄ alkyl or R^{c} and R^{d} may together with the carbon atom to which they are attached form a C₃₋₈ cycloalkyl group, and p and q independently represent an integer from 0 to 5 wherein p + q is an integer from 0 to 5;
and salts and solvates thereof;
with the provisos that;
the compound of formula (I) is not a compound of formula (I)^{a}: 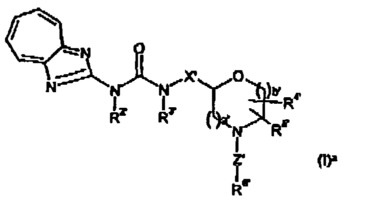 wherein R² represents hydrogen or lower alkyl (specifically C₁₋₄ alkyl); R^{3'} represents hydrogen; X' represents methylene or ethylene; a' and b' both represent 1; R^{4'} and R^{5'} both represent hydrogen; and wherein the moiety Z'-R^{6'} represents halobenzyl, and;
the compound of formula (I) is not a compound of formula (I)^{b}: 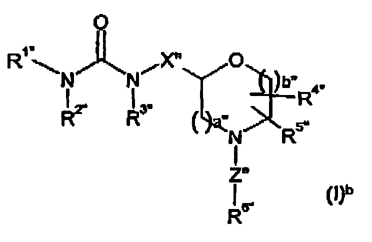 wherein R^{1"} represents a hydrogen atom, a C₁₋₆ alkyl group, a C₃₋₆ cycloalkyl group, a C₃₋₆ cycloalkylC₁₋₄ alkyl group, an aryl group or an arylC₁₋₄ alkyl group (particularly wherein aryl represents phenyl or naphthyl) in which the aryl moiety of the aryl group or arylC₁₋₄ alkyl group may be optionally substituted with a halogen atom, a C₁₋₆ alkyl group, a C₁₋₆ alkoxy group, a C₁₋₆ alkoxycarbonyl group or an amino group; R^{2"} represents hydrogen; R^{3"} represents hydrogen or C₁₋₆ alkyl; X" represents methylene; a" and b" both represent 1; R^{4"} and R^{5"} both represent hydrogen; and wherein the moiety -Z^{"}-R^{6"} represents a C₁₋₆ alkyl group, an arylC₁₋₄ alkyl group (particularly wherein aryl represents phenyl or naphthyl), a heteroarylC₁₋₄ alkyl group (particularly wherein heteroaryl represents 2-pyridyl, 3-pyridyl, 4-pyridyl or 1H-indol-3-yl), an aryloxyC₂₋₅ alkyl group or a pyrrolidinylcarbonylC₁₋₄ alkyl group in which the aryl moiety of the said groups may be optionally substituted with a halogen atom, a C₁₋₆ alkyl group, a C₁₋₆ alkoxy group, a C₁₋₆ alkoxycarbonyl group or an amino group.

Compounds of formula (I) possess a chiral carbon atom at the position marked"*" and may therefore exist as enantiomers.

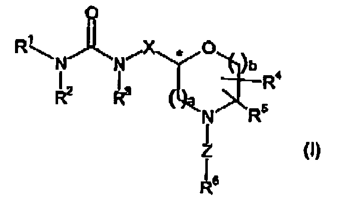

PCT/GB01/04350 (WO 02/026723) also discloses a process for the preparation of compounds of formula (I) wherein enantiomers thereof may be prepared by a combination of an achiral synthesis with a resolution step. Examples of such a resolution step are preparative chiral high performance liquid chromatography (preparative chiral HPLC) and the fractional crystallisation of diastersoisomeric salts. In particular, it is disclosed in PCT/GB01/04350 (WO 02/026723) that an enantiomer of a compound of formula (I) may be prepared by the resolution of a racemic modification of a compound of formula (III) 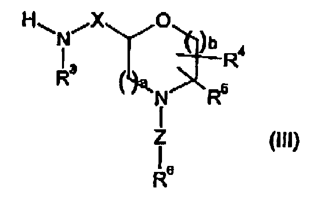 wherein;
R³, a, b, R⁴, R⁶, Z, and R⁶ are as defined In formula (I) above; by fractional crystallisation of a diastereisomeric salt thereof, followed by fraction of the resolved enantiomer of the compound of formula (III) with a compound of formula (X) to give a compound of formula (IV) 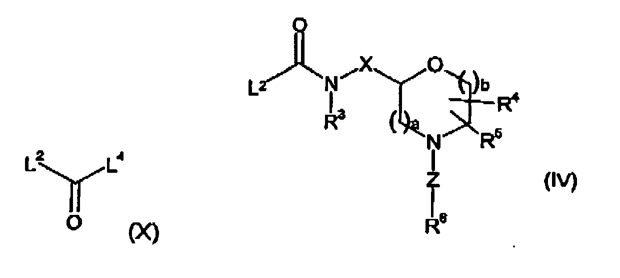
wherein;
L² and L⁴ are leaving groups, and R³, a, b, R⁴, R⁵, Z, and R⁶ are as defined in formula (I) above;
   followed by reaction of a compound of formula (IV) with a compound of formula (V) 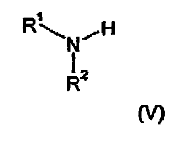 wherein;

R¹ and R² are as defined in formula (I) above; to give a compound of formula (I).

Alternative processes for preparing an enantiomer of certain compounds of formula (I), being of formula (IA) 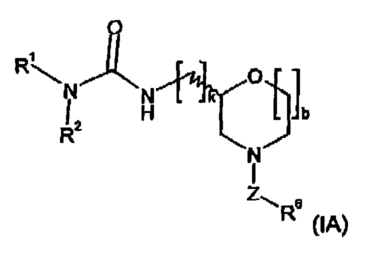 wherein;
R¹, R², b, Z, and R⁶ are as defined for formula (I), and;
k is 1 or 2;
and salts and solvates thereof has now been discovered, with the provisos that; the compound of formula (IA) is not a compound of formula (I)^{a}: 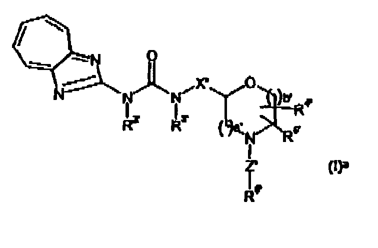 wherein R^{2'} represents hydrogen or lower alkyl (specifically C₁₋₄ alkyl); R^{3'} represents hydrogen; X' represents methylene or ethylene; a' and b' both represent 1; R^{4'} and R^{5'} both represent hydrogen; and wherein the moiety -Z'-R^{6'} represents halobenzyl, and;
the compound of formula (IA) is not a compound of formula (I)^{b}: 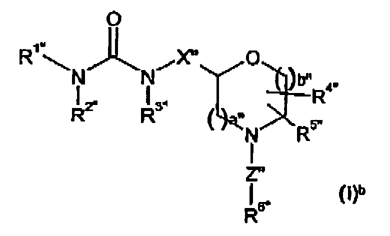 wherein R^{1"} represents a hydrogen atom, a C₁₋₆ alkyl group, a C₃₋₆ cycloalkyl group, a C₃₋₆ cycloalkylC₁₋₄ alkyl group, an aryl group or an arylC₁₋₄ alkyl group (particularly wherein aryl represents phenyl or naphthyl) in which the aryl moiety of the aryl group or arylC₁₋₄ alkyl group may be optionally substituted with a halogen atom, a C₁₋₆ alkyl group, a C₁₋₆ alkoxy group, a C₁₋₆ alkoxycarbonyl group or an amino group; R^{2"} represents hydrogen: R^{3"} represents hydrogen or C₁₋₆ alkyl; X" represents methylene; a" and b" both represent 1; R^{4"} and R^{5"} both represent hydrogen; and wherein the moiety -Z"-R^{6"} represents a C₁₋₆ alkyl group, an arylC₁₋₄ alkyl group (particularly wherein aryl represents phenyl or naphthyl), a heteroarylC₁₋₄ alkyl group (particularly wherein heteroaryl represents 2-pyridyl, 3-pyridyl, 4-pyridyl or 1H-indol-3-yl), an aryloxyC₂₋₅ alkyl group or a pyrrolidinylcarbonylC₁₋₄ alkyl group In which the aryl moiety of the said groups may be optionally substituted with a halogen atom, a C₁₋₆ alkyl group, a C₁₋₆ alkoxy group, a C₁₋₆ alkoxycarbonyl group or an amino group.

Co-pending International Patent Application publication WO 02/26722 (Glaxo Group Limited) discloses certain compounds of formula (Ip) 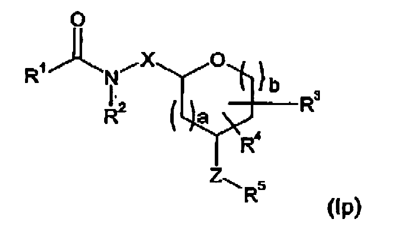 and processes for their preparation, including processes for the preparation of enentiomers of compounds of formula (Ip).

These processes involve the chiral synthesis of certain compounds of formula (III).

Accordingly, in a first aspect, there is provided a process for the preparation of a compound of formula (IIIA) 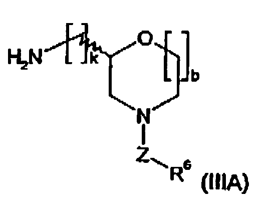 or a salt thereof;
wherein;
Z represents a bond, CO, SO₂, CR¹⁰R⁷(CH₂)ₙ, (CH₂)ₙCR¹⁰R⁷, CHR⁷(CH₂)ₙO, CHR⁷(CH₂)ₙS, CHR⁷(CH₂)ₙOCO, CHR⁷(CH₂)ₙCO, COCHR⁷(CH₂)ₙ or SO₂CHR⁷(CH₂)ₙ;
R⁶ represents C₁₋₆ alkyl, C₂₋₆ alkenyl, aryl, heteroaryl, aryl-C₂₋₆ alkenyl-, -CN or a group of formula -Y²-J³;
R⁷ represents hydrogen, C₁₋₄ alkyl, CONR⁸R⁹ or COOC₁₋₆ alkyl;
a and b represent 1 or 2, such that a+b represents 2 or 3;
n represents an integer from 0 to 4;
J³ represents a moiety of formula (K): 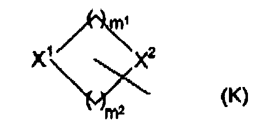
wherein X¹ represents oxygen, NR¹¹ or sulphur, X² represents CH₂, oxygen, NR¹² or sulphur, m¹ represents an integer from 1 to 3 and m² represents an integer from 1 to 3, provided that m¹+m² is in the range from 3 to 5, also provided that when both X¹ and X² represent oxygen, NR¹¹, NR¹² or sulphur, m¹ and m² must both not equal less than 2, wherein K is optionally substituted by one or more (eg. 1 or 2) -Y³-aryl, -Y³- heteroaryl, -Y³-CO-aryl, -COC₃₋₈ cycloalkyl, -Y³-CO-heteroaryl, -C₁₋₆ alkyl, -Y³-COOC₁₋₆ alkyl, -Y³-COC₁₋₆ alkyl, -Y³-W, -Y³-CO-W, -Y³-NR¹⁵R¹⁶, -Y³-CONR¹⁵R¹⁶, hydroxy, oxo, -Y³-SO₂NR¹⁵R¹⁶, -Y³-SO₂C₁₋₆ alkyl, -Y³-SO₂aryl, -Y³-SO₂heteroaryl, -Y³-NR¹³C₁₋₆ alkyl, -Y³-NR¹³SO₂C₁₋₆ alkyl, -Y³-NR¹³CONR¹⁵R¹⁶, -Y³-NR¹³COOR¹⁴ or -Y³-OCONR¹⁵R¹⁶ groups, and is optionally fused to a monocyclic aryl or heteroaryl ring;
R⁸, R⁹, R¹⁰, R¹¹, R¹², R¹³ and R¹⁴ independently represent hydrogen or C₁₋₆ alkyl;
R¹⁵ and R¹⁶ independently represent hydrogen or C₁₋₆ alkyl or R¹⁵ and R¹⁶ together with the nitrogen atom to which they are attached may form a morpholine, piperidine or pyrrolidine ring;
R¹⁷ and R¹⁸ independently represent hydrogen or C₁₋₆ alkyl;
W represents a saturated or unsaturated, non-aromatic 5-7 membered ring containing between 1 and 3 heteroatoms selected from nitrogen, oxygen or sulphur, optionally substituted with one or more C₁₋₆ alkyl, halogen or hydroxy groups;
Y¹, Y² and Y³ independently represent a bond or a group of formula - (CH₂)ₚCR^{c}R^{d}(CH₂)_{q}- wherein R^{c} and R^{d} independently represent hydrogen or C₁₋₄ alkyl or R^{c} and R^{d} may together with the carbon atom to which they are attached form a C₃₋₈ cycloalkyl group, and p and q independently represent an integer from 0 to 5 wherein p + q is an integer from 0 to 5;, and;
k is 1 or 2;
which process comprises the reaction of a compound of formula (XX) 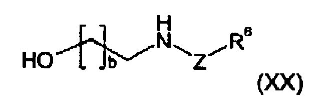 wherein;
b, Z, and R⁶ are as defined for formula (IIIA);
with an enantiomer of a compound of formula (XXI), under Mitsonobu conditions, 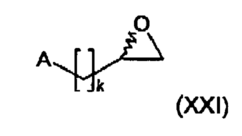
wherein;
A is a protected amino group and k is 1 or 2; followed by deprotection of the amino group to give a compound of formula (IIIA).

Suitable protecting groups for amines include phthalimido.

The compound of formula (IIIA) is typically prepared from the compounds of formulae (XX) and an enantiomer of a compound (XXI) under the Mitsonobu conditions as follows:

Typically, a mixture of the compound of formula (XX) and an enantiomer of a compound of formula (XXI) In a suitable solvent, such as tetrahydrofuran or toluene, is stirred, suitably for 2-36 hours at a suitable temperature, suitably the reflux temperature of the mixture, under an inert atmosphere, suitably an atmosphere of nitrogen. Further solvent, suitably toluene or tetrahydrofuran, is then added and the mixture cooled, suitably to 0-40°C. A phosphine, suitably triphenyl phosphine, is added and the mixture stirred until all the solid is dissolved. An azodicarboxylate, suitably diisopropylazodicarboxylate, is then added over a period of time, suitably 5-120 min, while maintaining the temperature at <40°C. The mixture is stirred at a suitable temperature, suitably 20-40°C. If necessary, further phosphine and azodicarboxylate reagents can be added. After a further period, the reaction mixture is concentrated to low volume. A suitable alcohol, suitably methanol or iso-propyl alcohol, is added and the concentration step repeated. This may be repeated as necessary. Further alcohol is then added and the mixture may be heated to a temperature suitably between 55-75°C. After a suitable period, suitably 20-45 minutes, the resultant slurry is cooled, suitably to 15-25°C, and then allowed to stand, suitably for 1.5-3 hours, after which time the product is isolated by filtration. The filter bed is washed with more alcohol and then dried *in vacuo* at 35-45°C to yield the protected compound of formula (IIIA).

The protected compound of formula (IIIA) may be deprotected to yield the compound of formula (IIIA) using standard conditions suitable for the removal of the particular protecting group, for example those conditions described in P J. Kocienski, Protecting Groups, (1994), Thieme*.*

The compounds of formulae (XX) and the enantiomers of a compound of formula (XXI) and (XXII) are known, commercially available compounds, or may be prepared by analogy with known procedures, for examples those disclosed in standard reference texts of synthetic methodology such as J. March, Advanced Organic Chemistry, 3rd Edition (1985), Wiley Interscience*.*

The compound of formula (IIIA) is known and is disclosed in J. Med. Chem., 1991, 34(2), 616-624*.*

Compounds of formula (IA) may then be prepared from compounds of formula (IIIA) as follows:
The compound of formula (IIIA) is reacted with a compound of formula (XA) 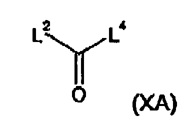
wherein;
L² and L⁴ represent leaving groups wherein L² and L⁴ are the same or L⁴ represents a leaving group which is more labile than L², to form a compound of formula (IVA) 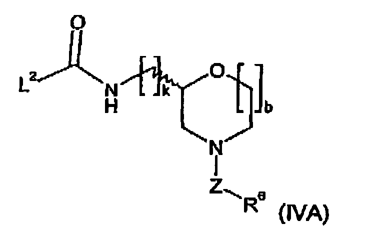
wherein;
L², k, b, Z, and R⁶ are as hereinbefore defined.

The compound of formula (IVA) is in turn is reacted with with a compound of formula (VA)

R¹-NHR² (VA)

wherein R¹ and R² are as defined in formula (I) above, to give a compound of formula (IA).

Compounds of formulae (XA), and (VA) are also known, commercially available compounds, or may be prepared by analogy with known procedures, for examples those disclosed in standard reference texts of synthetic methodology such as J. March, Advanced Organic Chemistry, 3rd Edition (1985), Wiley Interscience*.*

Suitable salts of the compounds of formula (IIIA) are those which may be 5 useful in terms of isolation or handling of the compound of formula (IIIA) or those which may be useful in the preparation of compounds of formula (IA) and physiologically acceptable salts thereof. If appropriate, add addition salts may be derived from inorganic or organic acids, for example tartrates, hydrochlorides, hydrobromides, sulphates, phosphates, acetates, benzoates, citrates, succinates, lactates, tartrates, fumarates, maleates, 1-hydroxy-2-naphthoates, palmoates, methanesulphonates, formates or trifluoroacetates. Salts of the compounds of formula (IIIA) may be prepared by procedures well known to those skilled in the art.

It is considered that the compound of formula (IIIB) is novel.

Accordingly, in an additional aspect, there is provided a compound of formula (IIIB) or a salt thereof.

Suitable salts of the compounds of the invention are those which may be useful In terms of isolation or handling of the compounds of the invention, If appropriate, acid addition salts may be derived from inorganic or organic adds, for example tartrates, hydrochlorides, hydrobromides, sulphates, phosphates, acetates, benzoates, citrates, succinates, lactates, tartrates, fumarates, maleates, 1-hydroxy-2-naphthoates, palmoates, methanesulphonates, formates or trifluoroacetates. Salts of the compounds of the invention may be prepared by procedures well known to those skilled in the art.

A compound of formula (IA) may be prepared from a compound of formula (IIIA) as follows.

Typically, a compound of formula (IIIA) in a suitable first solvent is reacted with N,N'-carbonyldiimidazole in the same solvent at reduced temperature, suitably a temperature In the range -10-20°C over a suitable period of time, for example 5-60 minutes. Suitable solvents include tetrahydrofuran, dichloromethane, C₃₋₄ alkanol, isopropyl acetate, N-methylpyrrolidinone and N,N-dimethylformamide. The mixture is warmed to a suitable temperature, suitably 5-30°C and held at this temperature for a suitable period of time, for example 10-60 minutes. The compound of formula (VA) is then added, the mixture heated to a suitable elevated temperature, for example a temperature in the range 40-65°C, and stirred for a suitable period of time, for example 60-360 minutes. The reaction is then cooled to a suitable temperature, and a suitable second solvent, for example isopropyl acetate, added, followed by a aqueous solution of a suitable acidic salt, such as potassium dihydrogen phosphate or acetic add. The solution is clarified if necessary, the lower aqueous layer removed and the upper organic layer washed with further acidic salt solution, followed by water. The organic phase is distilled at atmospheric pressure to remove the first solvent and leave a slurry or solution of the compound of formula (IA) in the second solvent. The compound of formula (IA) may be isolated by filtration or evaporation of the solvent as appropriate.

A compound of formula (Ip) may be prepared from a compound of formula (IIIA) and a compound of formula R'-COOH wherein R¹ is as defined for formula (Ip) as follows.

Typically, a solution of a compound of formula R¹-COOH in N,N-dimethylformamide in an inert atmosphere, suitably an atmosphere of nitrogen, is treated with O-(7-azabenzotriazol-1-yl)-N,N,N'N'-tetramethylammonium hexafluorophosphate and N,N-dilsopropylethylamine followed by a solution of a compound of formula (IIIA) in a suitable solvent, for example N,N-dimethylformamide, and the mixture stirred at 18-30°C for 2-8 hours. The solvent is removed in vacuo and the residue dissolved in a suitable solvent, for example ethyl acetate. The solution is washed with 10% aqueous citric acid, brine, saturated aqueous sodium hydrogen carbonate, and brine dried over magnesium sulphate and evaporated in vacuo.

Suitable salts of the compounds of formulae (IA) and (Ip) include physiologically-acceptable salts and salts which may not be physiologically acceptable but may be useful in the preparation of compounds of formulae (IA) and (Ip) and physiologically acceptable salts thereof. If appropriate, add addition salts may be derived from Inorganic or organic acids, for example hydrochlorides, hydrobromides, sulphates, phosphates, acetates, benzoates, citrates, succinates, lactates, tartrates, fumarates, maleates, 1-hydroxy-2-naphthoates, palmoates, methanesulphonates, formates or trifluoroacetates. Examples of solvates include hydrates.

Salts and solvates of the compounds of formulae (IA) and (Ip)may be prepared by procedures well known to those skilled in the art.

Suitable protecting groups In any of the above mentioned reactions are those used conventionally in the art. The methods of formation and removal of such protecting groups are those conventional methods appropriate to the molecule being protected, for example those methods discussed In standard reference texts of synthetic methodology such as P J Koclenski, Protecting Groups, (1994*), Thieme.*

Suitably, the variable R¹ of compounds of formulae (IA) and (VA) represents C₁₋₆ alkyl, C₂₋₆ alkenyl, C₂₋₆ alkynyl, aryl-Y¹-, heteroaryl-Y¹-, aryl-(O)ₜ- aryl-Y¹-, aryl-(O)ₜ-heteroaryl-Y¹-, heteroaryl-(O)ₜ-aryl-Y¹-, heteroaryl-(O)ₜ-heteroaryl-Y¹-, aryl- SO₂Y¹-, C₁₋₆ alkyl-G-Y¹-, J¹-SO₂Y¹-, R¹⁷O(CO)-C₂₋₆ alkenyl-Y¹-, C₂₋₆ alkynyl-Y¹-, C₂₋₆ alkenyl-Y¹-, aryl-O-Y¹-, heteroaryl-O-Y¹-, C₁₋₆ alkyl-SO₂-Y¹-, M-Y¹-, J¹-Y¹-, J¹-CO-Y¹-, aryl-CO-Y¹- or C₃₋₈ cycloalkyl-Y¹- or C₃₋₈ cycloalkenyl-Y¹-, which C₂₋₆ alkynyl and C₂₋₆ alkynyl-Y¹ may be optionally substituted with a -OR¹⁷ group and which cycloalkyl or cycloalkenyl may be optionally substituted by one or more hydroxyl or C₁₋₆ alkyl groups;
J¹, J² and J³ Independently represent a moiety of formula (K): 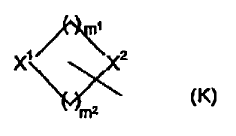 wherein X¹ represents oxygen, NR¹¹ or sulphur, X² represents CH₂, oxygen, NR¹² or sulphur, m¹ represents an integer from 1 to 3 and m² represents an integer from 1 to 3, provided that m¹+m² is in the range from 3 to 5, also provided that when both X¹ and X² represent oxygen, NR¹¹, NR¹² or sulphur, m¹ and m² must both not equal less than 2, wherein K is optionally substituted by one or more (eg. 1 or 2) -Y³-aryl, -Y³-heteroaryl, -Y³-CO-aryl, -Y³-CO-heteroaryl, -C₁₋₆ alkyl, -Y³-COOC₁₋₆ alkyl, -Y³-COC₁₋₆ alkyl, -Y³-W, Y³-CO-W, -Y³-NR¹⁵R¹⁶, - Y³-CONR¹⁵R¹⁶, hydroxy, oxo, -Y³-SO₂NR¹⁵R¹⁶, -Y³-SO₂C₁₋₆ alkyl, -Y³-SO₂aryl,-Y³-SO₂heteroaryl, -Y³-NR¹³C₁₋₆ alkyl, -Y³-NR¹³SO₂C₁₋₆ alkyl, -Y³-NR¹³CONR¹⁵R¹⁶, -Y³-NR¹³COOR¹⁴ or -Y³-OCONR¹⁵R¹⁶ groups, and is optionally fused to a monocyclic aryl or heteroaryl ring.

Suitably, the variable R² of compounds of formulae (IA) and (VA) represents hydrogen or C₁₋₆ alkyl.

More suitably, the variable R¹ of compounds of formulae (IA) and (VA) represents C₁₋₆ alkyl, C₂₋₆ alkenyl, C₂₋₆ alkynyl, aryl-Y¹-, heteroaryl-Y¹-, aryl-(O)ₜ-aryl-Y¹-, aryl-(O)ₜ-heteroaryl-Y¹-, heteroaryl-(O)ₜ-aryl-Y¹-, heteroaryl-(O)ₜ-heteroaryl-Y¹-, C₂₋₆ alkenyl-Y¹-, aryl-O-Y¹-, heteroaryl-O-Y¹-, C₁₋₆ alkyl-SO₂-Y¹-, M-Y¹-, -Y¹-J¹, -Y¹-CO-J¹ or C₃₋₈ cycloalkyl-Y¹- or C₃₋₈ cycloalkenyl-Y¹-, which cycloalkyl or cycloalkenyl may be optionally substituted by one or more hydroxyl or C₁₋₆ alkyl groups;
J¹, J² and J³ Independently represent a moiety of formula (K): 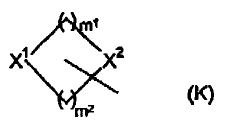
wherein X¹ represents oxygen, nitrogen, NR¹¹ or sulphur, X² represents CH₂, oxygen, nitrogen, NR¹² or sulphur, m¹ represents an Integer from 1 to 3, m² represents an integer from 1 to 3, provided that m¹+m² is in the range from 3 to 5, also provided that when X² represents oxygen, nitrogen, NR¹² or sulphur, m¹ and m² must both not equal less than 2, wherein K is optionally substituted by one or more (eg. 1 or 2) -Y³-aryl, -Y³-heteroaryl, -Y³-CO-aryl, -Y³-CO-heteroaryl, -C₁₋₆ alkyl, -Y³-COOC₁₋₆ alkyl, -Y³-COC₁₋₆ alkyl, -Y³-W, -Y³-CO-W, -Y³-NR¹⁵R¹⁶, - Y³-CONR¹⁵R¹⁶, hydroxy, oxo, -Y³-SO₂NR¹⁵R¹⁶, -Y³-SO₂C₁₋₆ alkyl, -Y³-SO₂aryl, - Y³-SO₂heteroaryl, -Y³-NR¹³C₁₋₆ alkyl, -Y³-NR¹³SO₂C₁₋₆ alkyl, -Y³-NR¹³CONR¹⁵R¹⁶, -Y³-NR¹³COOR¹⁴ or -Y³-OCONR¹⁵R¹⁶ groups, and is optionally fused to a monocyclic aryl or heteroaryl ring.

More suitably, the variable R² of compounds of formulae (IA) and (VA) represents hydrogen or C₁₋₆ alkyl.

Preferred values of Z for compounds of formulae (IIIA), (XX), and (IA) are those wherein Z represents a bond, CO, SO₂, CR¹⁰R⁷(CH₂)ₙ, CHR⁷(CH₂)ₙO, CHR⁷(CH₂)ₙS, CHR⁷(CH₂)ₙOCO, or CHR⁷(CH₂)ₙCO.

References to 'aryl' include references to monocyclic carbocyclic aromatic rings (eg. phenyl) and bicyclic carbocyclic aromatic rings (e.g. naphthyl) and references to 'heteroaryl' include references to mono- and bicyclic heterocyclic aromatic rings containing 1-3 hetero atoms selected from nitrogen, oxygen and sulphur. References to 'heteroaryl' may also be extended to include references to mono- and bicyclic heterocyclic aromatic rings containing 4 hetero atoms selected from nitrogen, oxygen and sulphur. Examples of monocyclic heterocyclic aromatic rings include e.g. pyridinyl, pyrimidinyl, thiophenyl, furanyl, pyrrolyl, oxazolyl, isoxazolyl, oxadiazolyl, thiazolyl, thiadiazolyl. Further examples of monocyclic heterocyclic aromatic rings include pyrazinyl, tetrazolyl or imidazolyl. Examples of bicyclic heterocyclic aromatic rings include eg. quinolinyl or indolyl. Further examples of bicyclic heterocyclic aromatic rings include benzimidazolyl. Yet further examples of bicyclic heterocyclic aromatic rings include dihydrobenzofuranyl and pyrrolopyridinyl, Carbocyclic and heterocyclic aromatic rings may be optionally substituted, e.g. by one or more C₁₋₆ alkyl, C₂₋₆ alkenyl, halogen, C₁₋₆alkoxy, cyano, hydroxy, nitro, amino, W, -N(CH₃)₂,-NHCOC₁₋₆ alkyl, -OCF₃, -CF₃, -COOC₁₋₈ alkyl, -OCHF₂, -SCF₃, -CONR¹⁹R²⁰, - SO₂NR¹⁹R²⁰ (wherein R¹⁹ and R²⁰ independently represent hydrogen, C₁₋₆ alkyl or C₃₋₈ cycloalkyl), -NHSO₂CH₃, -SO₂CH₃ or -SCH₃ groups. A further substituent of carbocyclic and heterocyclic aromatic rings may be -COOH. Yet further substituents of carbocyclic and heterocyclic aromatic rings may be -CH₂N(CH₃)₂ or one or more -SH groups, wherein it will be appreciated that said group may tautomerise to form an =S group,

Examples of group M include tetrahydronaphthalenyl,

Examples of group W include piperidinyl, pyrrolidinyl, morpholinyl and piperazinyl which may be optionally substituted with one or more C₁₋₆ alkyl, halogen, or hydroxy groups.

Examples of group J¹ include N-(COOCH₂CH₃)-piperidin-4-yl, N-(CH₃)-piperidin-4-yl, N-(COCH₃)-piperidin-4-yl, pyrrolidin-1-yl, tetrahydropyran-4-yl or N-morpholinyl. Further examples of group J¹ include N-(cyclopropylcarbonyl)-piperidin-4-yl, N-(methylsulphonyl)-piperidin-4-yl, thiopyranyl and tetrahydrothienyl.

Examples of group J² include (4-phenyl)-piperidin-1-yl, (4-COOCH₂CH₃)-piperazin-1-yl, (2-(3-hydroxy-pyrrolidin-1-yl-methyl))piperidin-1-yl, N-morpholinyl, (4-N(CH₃)₂)-piperidin-1-yl, (4-(3-fluorophenyl))-piperazin-1-yl, (4-(4-fluorophenyl))-piperazin-1-yl, (4-pyrimidinyl)-piperazin-1-yl, (4-CH₃)-piperazin-1-yl, (4-CONH₂)-piperidin-1-yl, (3,3-dimethyl)-piperidin-1-yl, (4-COCH₃)-piperazin-1-yl, (4-(1-pyrrolidinyl-carbonylmethyl))-piperazin-1-yl, (4-hydroxy)-piperidin-1-yl, (4-methyl)-piperidin-1-yl, (4-(2-furanyl-carbonyl))-piperazin-1-yl, (4-benzyl)-piperazin-1-yl or (3-CH₃SO₂CH₂-)-morpholin-1-yl. Further examples of group J² include thiomorpholinyl, pyrrolidinyl and benzazepinyl.

Examples of group J³ include indolinyl, which may be optionally substituted.

References to alkyl include references to both straight chain and branched chain aliphatic isomers of the corresponding alkyl. It will be appreciated that references to alkylene and alkoxy shall be interpreted similarly. References to C₃₋₈ cycloalkyl include references to all alicyclic (including branched) isomers of the corresponding alkyl.

Preferably, R¹ represents C₁₋₆ alkyl (particularly propyl), C₂₋₆ alkenyl (particularly wherein said C₂₋₆ alkenyl is substituted by one or more -COOR¹⁷ groups, eg. -HC=CH-COOH), C₂₋₆ alkynyl, aryl-Y¹-, heteroaryl-Y¹- (particularly wherein heteroaryl represents thiazolyl, indolyl, furanyl, dihydrobenzofuran, oxoimidazolyl, isoxazolyl, thienyl, thioxodihydroimidazolyl, tetrazolyl, pyrazinyl, pyrrolopyridinyl), aryl(O)-aryl-Y¹-, aryl-(O)-heteroaryl-Y¹- (particularly wherein aryl represents phenyl and heteroaryl represents thiadiazolyl, pyrazolyl or isoxazolyl), heteroaryl-(O)-aryl-Y¹-, heteroaryl-(O)-heteroaryl-Y¹-, C₂₋₆ alkenyl-Y¹-, aryl-O-Y¹- (particularly wherein aryl represents phenyl), heteroaryl-O-Y¹-, C₁₋₆ alkyl-SO₂-Y¹- (particularly wherein C₁₋₆ alkyl represents ethyl, propyl, -CH(CH₃)₂ or -C(CH₃)₃),M-Y¹-, J¹-Y¹-, J¹-CO-Y¹-, aryl-SO₂-Y¹-, C₁₋₆ alkyl-G-Y¹- (particularly wherein C₁₋₈ alkyl represents methyl and G represents -NR¹⁸CO-, -CONR¹⁸-, - NR¹⁸SO₂- or -SO₂NR¹⁸-), heteroaryl-G-aryl-Y¹- (particularly wherein aryl represents phenyl and heteroaryl represents thiazolyl and G represents - NR¹⁸SO₂-), J¹-SO₂-Y¹- (particularly wherein J¹ represents 1-pyrrolidinyl). R¹⁷(CO)-C₂₋₆ alkenyl-Y¹-, R¹⁷NHCO-Y¹- (particularly wherein R¹⁷ represents hydrogen), C₂₋₆ alkynyl-Y¹- (particularly -C=CH or wherein said C₂₋₆ alkynyl is substituted with a -OR¹⁷ group, eg. HOCH₂-C≡C-), aryl-CO-Y¹- (particularly wherein aryl represents phenyl), C₃₋₈ cycloalkyl-Y¹- or C₃₋₈ cycloalkenyl-Y¹-, which cycloalkyl or cycloalkenyl may be optionally substituted by one or more hydroxyl or C₁₋₆ alkyl groups and which C₂₋₆ alkynyl-Y¹- may be optionally substituted with a -OR¹⁷ group.

More preferred R¹ groups include C₁₋₆ alkyl, C₂₋₆ alkenyl, C₂₋₆ alkynyl, aryl-5 Y¹-, heteroaryl-Y¹-, aryl-(O)ₜ-aryl-Y¹-, aryl-(O)ₜ-heteroaryl-Y¹-, hetoroaryl-(O)ₜ-aryl-Y¹-, heteroaryl-(O)ₜ-hetercaryl-Y¹-, C₂₋₆ alkenyl-Y¹-, aryl-O-Y¹-, heteroaryl-O-Y¹-, C₁₋₆, alkyl-SO₂-Y¹-, M-Y¹-, J¹-Y¹-, J¹-CO-Y¹- or C₃₋₈ cydoalkyl-Y'- or C₃₋₈ cycloalkeny-Y¹-, which cycloalkyl or cycloalkenyl may be optionally substituted by one or more hydroxyl or C₁₋₆ alkyl groups.

Yet more preferably, R¹ represents aryl-Y¹-, heteroaryl-Y¹, aryl-(O)ₜ-aryl-Y¹-, C₃₋₈, cycloalkyl-Y¹-, C₂₋₆ alkenyl-Y¹- or C₁₋₆ alkyl-SO₂-Y¹- especially wherein aryl represents phenyl or naphthyl optionally substituted by one or more C₁₋₆ alkyl (especially methyl), halogen (especially chlorine, fluorine and bromine), CH₃O-, CH₃S-, F₂CHO-, CH₃OC(O)-, -CN, -CF₃, CF₃-S-, CF₃-O-, or (CH₃)₂N-, groups, and wherein heteroaryl represents pyridinyl optionally substituted by one or more halogen atoms (especially chlorine) and wherein cycloalkyl represents cyclohexyl. Further preferred substituents of phenyl include -NHCOCH₃ and -CONH₂, Yet further preferred substituents of phenyl include -SO₂NH₂, - CONHCH₃, -OCH(CH₃)₂, -OC(CH₃)₃, -COOH, -CON(CH₃)₂, -SO₂CH₃, - CONHCH₂CH₃, -CONHcyclopropyl and -SO₂NHcyclopropyl. Also preferably, R¹ represents C₂₋₆ alkynyl-Y¹-, A series of particularly preferred compounds are those wherein R¹ represents aryl-Y¹- wherein aryl represents phenyl optionally substituted by one or more C₁₋₆ alkyl (especially methyl), halogen (especially chlorine, fluorine and bromine), CH₃O-, CH₃S-, F₂CHO-, CH₃OC(O)-, -CN or-CF₃ groups. Further most preferred substituents of phenyl include -NHCOCH₃ and -CONH₂. A yet further most preferred substituent of phenyl includes SO₂NH₂. Most preferably, R¹ will also represent C₂₋₆ alkenyl-Y¹- (particularly CH₂=CH-Y¹-), C₃₋₆ cycloalkyl-Y¹- (particularly cyclohexyl-Y¹-) and C₁₋₆alkyl-SO₂Y¹- (particularly CH₃SO₇-Y¹-). Also most preferably, R¹ represents C₂₋₆ alkynyl-Y¹- (particularly HC≡C-Y¹).

Especially preferred R¹ groups are aryl-Y¹- and heteroaryl-Y¹-, most especially wherein aryl represents phenyl and heteroaryl represents a 5 membered monocyclic heterocyclic aromatic ring (most particularly tetrazolyl) each of which may be optionally substituted as indicated above.

Preferred substituents of heteroaryl include -CH₃, -CONH₂, -CH₂N(CH₃)₂, halogen (particularly chlorine), -OCH₃, -COOCH₃ and -NH₂.

Most especially preferred compounds are those wherein R¹ represents phenyl-Y¹- which phenyl is substituted with a -CONH₂ or -CONHCH₃ group, preferably -CONH₂, and tetrazolyl-Y¹- which tetrazolyl is substituted with a methyl group.

Preferably, Y¹ represents a bond or C₁₋₈ alkylene, more preferably a bond, methylene or ethylene, propylene, -C(CH₃)₂-or -CH(CH₃)-, particularly a bond, methylene or ethylene, most preferably a bond or methylene, especially methylene.

Preferably, Y² represents a bond.

Preferably, Y³ represents a bond.

Preferably, R² represents hydrogen, methyl or hydroxypropyl, more preferably hydrogen or methyl, especially hydrogen.

Also preferably, R¹ and R² together with the nitrogen atom to which they are attached form a group of formula J² wherein said nitrogen atom substitutes for either X¹ or X².

Preferably, R⁴ and R⁵ independently represent hydrogen or methyl. Most preferably, R⁴ and R⁵ represent hydrogen.

Preferably, Z represents a bond, CO, SO₂, CR¹⁰R⁷(CH₂)ₙ, CHR⁷(CH₂)ₙO, CHR⁷(CH₂)ₙS, CHR⁷(CH₂)ₙOCO or CHR⁷(CH₂)ₙCO.

More preferably, Z represents CO, CHR⁷(CH₂)ₙ, CHR⁷(CH₂)ₙO, CHR⁷(CH₂)ₙS, CHR⁷(CH₂)ₙOCO or CHR⁷(CH₂)ₙCO, especially CH₂CO, (CH₂)₂, (CH₂)₂S, (CH₂)₂O, (CH₂)₂OCO, (CH₂)₃CO, CO, CHR⁷, particularly CH₂, CHCH₃ or CH₂CO, most particularly CH₂ or CH₂CO, especially CH₂.

Preferably, R⁶ represents C₁₋₆ alkyl, C₂₋₆ alkenyl, CN, aryl, heteroaryl or a group of formula -Y²-J³, more preferably R⁶ represents phenyl (optionally substituted with one or more halogen, phenyl or C₂₋₆ alkenyl groups), naphthyl, C₁₋₆ alkyl, C₂₋₆ alkenyl, CN or a 5 membered aromatic heterocyclic ring containing 1 to 3 heteroatoms selected from O, N or S optionally substituted by halogen or C₁₋₆ alkyl. Especially, R⁶ represents phenyl (optionally substituted with one or more halogen (especially chlorine, fluorine or iodine), phenyl or 3-CH=CH₂ groups), naphthyl, indolinyl, methyl, -CH=CH₂, -CN or thiophenyl optionally substituted by halogen (especially chlorine), Most preferred R⁶ represents indolinyl (especially Indolin-1-yl) or else represents phenyl substituted by one or more halogen (eg. chlorine or fluorine) groups, particularly dichlorophenyl, 3-chlorophenyl, 5-chlorothiophenyl, 4-fluorophenyl and 3,4-difluorophenyl, most particularly dichlorophenyl, especially 3,4-dichlorophenyl.

Preferably, R⁷ represents hydrogen, methyl, COOC₁₋₆ alkyl or CONR⁸R⁹, more preferably hydrogen, COOC₁₋₆ alkyl or CONR⁸R⁹ most preferably hydrogen, COOEt or CONR⁸R⁹, especially hydrogen.

Preferably, R⁸ and R⁹ represent hydrogen.

Preferably, R¹⁰ represents hydrogen.

Preferably, R¹¹ and R¹² independently represent hydrogen or methyl.

Preferably, R¹³ and R¹⁴ independently represent hydrogen or methyl.

Preferably, R¹⁵ and R¹⁶ independently represent hydrogen or methyl or R¹⁵ and R¹⁶ together with the nitrogen atom to which they are attached may form a morpholine, piperidine or pyrrolidine ring, especially hydrogen or methyl.

Preferably, R¹⁷ represents hydrogen.

Preferably, R¹⁸ represents hydrogen.

Preferably, R¹⁹ and R²⁰ independently represent hydrogen, C₁₋₆ alkyl or C₃₋₈ cycloalkyl, especially hydrogen, cyclopropyl or methyl. Particularly, R¹⁹ and R²⁰ represent hydrogen.

Preferably, R^{c} represents hydrogen or methyl, particularly hydrogen.

Preferably. R^{d} represents hydrogen or methyl, particularly hydrogen.

Preferably, b represents 1.

Preferably, n represents 0,1 or 2.

Preferably, p + q equals an integer from 0 to 2, more preferably, p and q independently represent 0 or 1 such that p + q equals an integer from 0 to 1.

Preferably, t represents 0.

Preferably, W represents pyrrolidinyl or piperidinyl, especially pyrrolidinyl.

Preferably, X¹ represents oxygen, nitrogen or NR¹¹.

Preferably, X² represents CH₂, oxygen, nitrogen or NR¹².

Preferably, m¹ and m² independently represent an Integer from 1 to 2, such that m¹ + m² is in the range from 3 to 4.

Preferably, J¹ represents piperidinyl (particularly piperidin-4-yl) or tetrahydropyranyl (particularly tetrahydropyran-4-yl) optionally substituted by one or two -COOCH₂CH₃, -COOtBu, -CH₃, -COCH₃, -SO₂N(CH₃)₂, -SO₂CH₃, - COPhenyl or 3, 5-dimethylisoxazol-4-ylsulphonyl groups. Also preferably, J¹ represents morpholinyl, thiopyranyl or tetrahydrothienyl which may be optionally substituted as above (particularly dioxidotetrahydrothienyl).

Preferred substituents for J¹ include -CH₂-aryl (particularly wherein aryl represents phenyl optionally substituted with one or more halogen atoms, eg. dichlorophenyl), -COcyclopropyl or -Y³-SO₂heteroaryl (particularly wherein heteroaryl represents dimethylisoxazolyl).

Preferably, J² represents piperidinyl (particularly piperidin-1-yl), morpholinyl (particularly N-morpholinyl) or piperazinyl (particularly piperazin-1-yl) optionally substituted by one or two phenyl, -COOCH₂CH₃, -N(CH₃)₂, fluorophenyl, -CH₃, -CONH₂, -COCH₃, -CH₂CO-(N-pyrrolidinyl), hydroxy, -CO-(2-furan), benzyl or -CH₂SO₂CH₃. Preferably, J² also represents thiomorpholinyl, pyrrolidinyl or benzazepinyl optionally substituted In a similar manner.

Other preferred substituents for J² include halogen (particularly fluorine), -COOCH₂CH₃, -CO-furoyl, -SO₂CH₃, -pyridinyl-CH₃ or oxo groups.

Preferably, J³ represents indolinyl, particularly indolin-1-yl.

In a most preferred aspect, the variables R¹ and R² of compounds of formulae (IA) and (VA) represent 4-amidobenzyl or 2-methyltetrazol-5-ylmethyl, and hydrogen respectively; the variables b, Z, and R⁶ for the compounds of formulae (IIIA), (XX), (IVA), and (IA) represent 1, -CH₂-, and 3,4-dichlorophenyl respectively; and the variable k for the compounds of formulae (IIIA), (XXI), and (IA) represents 1.

Suitable salts of the compounds of formula (IA) include physiologically acceptable salts and salts which may not be physiologically acceptable but may be useful in the preparation of compounds of formula (I) and physiologically acceptable salts thereof. If appropriate, acid addition salts may be derived from inorganic or organic acids, for example hydrochlorides, hydrobromides, sulphates, phosphates, acetates, benzoates, citrates, succinates, lactates, tartrates, fumarates, maleates, 1-hydroxy-2-naphthoates, palmoates, methanesulphonates, formates or trifluoroacetates. Examples of solvates include hydrates. Salts and solvates of the compounds of formula (IA) may be prepared by procedures well known to those skilled in the art.

Throughout the specification and the claims which follow, unless the context requires otherwise, the word 'comprise', and variations such as 'comprises' and 'comprising', will be understood to imply the inclusion of a stated integer or step or group of integers but not to the exclusion of any other integer or step or group of integers or steps.

The following Examples illustrate the invention but do not limit it in any way,

### General experimental details

### NMR

Nuclear magnetic resonance (NMR) spectra were acquired using a Bruker DPX250 or DPX400 instrument

### LC/MS System A

The following Liquid Chromatography Mass Spectroscopy (LCMS) system was used: 3mm ABZ+PLUS (3.3cm x 4.6mm internal diameter) column, eluting with solvents: A - 0.1% formic acid + 0.077% w/v ammonium acetate in water, and B - 95:5 acetonitrile:water + 0,05%v/v formic acid, at a flow rate of 3ml per minute. The following gradient profile was used: 100% A for 0.7min; A + B mixtures, gradient profile 0 - 100% B over 3.5min; hold at 100%B for 1.1 min: return to 100% A over 0.2min.

### LC/MS System B

3µm Phenomenex Luna (50 x 2mm i.d.) column, eluting with solvents: A - 0.05% trifluoroacetic acid in water, B - 0.05% trifluoroacetic add in acetonitrile, at 40°C and at a flow rate of 1ml per minute. The following linear gradient was used: 0 to 95% B over 8 minutes.

### Analytical HPLC column, conditions and eluent

Reverse-phase high performance liquid chromatography was carried out using a Luna 3mm C18(2) (50 x 2.0mm i.d.) column eluting with solvents: A-100% water, 0.05% TFA; and B-100% acetonitrile, 0.05%TFA, at a flow rate of 2ml per minute, and at 60°C. The following gradient profile was used:0-95% B over 2.0min, return to 0% B over 0.01 min.

### Chiral analytical HPLC

(Chiralpak AD column, 4.6 x 250mm, eluent 50:50:0.1 MeOH: EtOH: Butylamine, flow rate 0.5ml/min, UV detection at 220nm), Rt 8.9min.

### Examples

### Reference Example 1: Preparation of [(2S)-4-(3,4-dichlorobenzyl)morpholin-2-yl]methylamine-enzyme method

To a solution of [4-(3,4-dichlorobenzyl)morpholin-2-yl]methylamine (6g) and ethyl octanoate (17.2ml) in *tert-*butyl methyl ether (60ml) was added enzyme Lipase PS-C "Amano" II (6g), under an atmosphere of nitrogen. The mixture was stirred at 200rpm and heated to 30°C. The mixture was stirred at 30°C for a further 6h. The enzyme was removed by vacuum filtration and washed with *tert-*butyl methyl ether. Deionised water (30ml) was added to the filtrate and the resultant bi-phasic solution was pH adjusted to pH 5.7 and the layers were separated. To the aqueous phase was added dichloromethane (30ml) and the resultant bi-phasic mixture was pH adjusted to pH 6.6. The aqueous phase was separated, diluted with dichloromethane and the pH was adjusted to >pH 9. The layers were separated and solvent was evaporated in vacuo to give the title compound as a yellow oil (2.1g, 99%a/a, 96%ee)
LC/MS (System A) Rₜ 1.77 min, Mass Spectrum *m*/*z* 275 [MH⁺].

### Example 2: Preparation of 2-{[(2R)-4-(3,4-dichlorobenzyl)morpholin-2-yl]methyl}-1H-isoindole-1,3(2H)-dione

A mixture of 2-[(3,4-dichlorobenzyl)amino]ethanol (2,038 g) and (S)-2-(oxiran-2-ylmethyl)-1H-isoindole-1,3(2H)-dione (N-(2,3-epoxypropyl)-phthalimide) (2.032g) in tetrahydrofuran (3.3ml) was stirred and heated at reflux under nitrogen. After 21.5h more tetrahydrofuran (12.5ml) was added and the mixture was cooled to 3°. Triphenyl phosphine (2.793g) was added and the mixture was stirred until all the solid had dissolved. Diisopropylazodicarboxylate (2.1 ml) was then added over 12min maintaining the temperature at <7°. After 2.25h the mixture was allowed to warm to 22°. After 5.3h more triphenylphosphine (121 mg) and diisopropylazodicarboxylate (0.09ml) were added. After 22.5h the reaction mixture was concentrated to near dryness. Propan-2-ol (12ml) was added and the concentration repeated, this was repeated once more. More propan-2-ol (12ml) was added and the mixture was heated to 70°. After 0.5h the slurry was cooled to 22° and then after a further 2h the product was collected. The bed was washed with propan-2-ol (2x4ml) and then dried *in vacuo* at 40° to give the title compound (2.622g).
NMR (DMSO d-6): 1.93δ (1H) d of d, J=11.0Hz, 8.8Hz; 2.10δ (1H) d of t, J=3.5Hz, 11.3Hz; 2.52δ (1H) broad d, J=11.3Hz; 2.77δ (1H) broad d, J=11.3Hz; 3.3 - 3.88 (7H) m; 7.318 (1H) d of d, J=8.2Hz, 1.9Hz; 7.55δ (1H) d, J=1.9Hz; 7.68δ (1H) d, J=8.2Hz; 7.86δ (4H) m.

### Preparation of [(2S)-4-(dichlorobenzyl)morpholin-2-yl]methylamine

A slurry of 2-{[(2*R*)4-(3,4-dichlorobenzyl)morpholin-2-yl]methyl}-1*H*-isoindole-1,3(2*H*)-dione (1.00g) in water(8.5ml) was heated to 75° and then treated dropwise with concentrated sulphuric acid (2.5ml). The mixture was then heated at reflux. After 23h the reaction mixture was cooled to 22° and then treated with dichloromethane (6ml). 880 Ammonia solution (7ml) was then added dropwise with cooling. More dichloromethane (10ml) was added. The aqueous phase was separated and extracted with more dichloromethane (10ml). The combined organic phase was washed with water (5ml) and then evaporated to dryness. The residue was reevaporated from DCM to give the title compound as an oil (662mg).
LC/MS (System A) R₁ 1.77 min. Mass Spectrum *m*/*z* 275 [MH⁺].

### Reference Example 3: Preparation of [(2S)-4-(3,4-dichlorobenzyl)morpholin-2-yl]methylamine - enzyme method

To a solution of [4-(3,4-dichlorobenzyl)morpholin-2-yl]methylamine (3g) and ethyl octanoate (5,8ml) in *tert*-butyl methyl ether (30ml) was added enzyme Upase PS-C "Amano" II (3g), under an atmosphere of nitrogen. The mixture was stirred at 200rpm and heated to 30°C. The mixture was stirred at 30°C for a further 6.5h. The enzyme was removed by vacuum filtration. To the filtrate was added de-ionised water (15ml). The resultant bi-phasic solution was pH adjusted to pH 5.5 and the layers were separated. To the aqueous phase was added DCM (15ml) and the resultant bi-phasic mixture was pH adjusted to pH 6.5. The layers were separated and solvent was evaporated in vacuo to give the title compound as a yellow oil (1.0g, 98.9%a/a, 94.8%ee)
LC/MS (System A) Rₜ 1.77 min, Mass Spectrum *m*/*z* 275 [MH⁺].

### Example 4: Preparation of 2-[[(2R)-4-(3,4-dichlorobenzyl)morpholin-2-yl]methyl]-1H-isoindole-1,3(2H)-dione

A mixture of 2-[(3,4-dichlorobenzyl)amino]ethanol (400g g) and (S)-2-(oxiran-2-ylmethyl)-1H-isoindole-1,3(2H)-dione (N-(2,3-epoxypropyl)-phthalimide) (399.6g) in toluene (1150ml) was stirred and heated at 103-107°C under nitrogen. After 22.5h the mixture was cooled to <60°C and charged with tetrahydrofuran (2800ml). Triphenyl phosphine (548g) was added and the mixture was stirred until all the solid had dissolved, then cooled to 5-9°C.
Diisopropylazodicarboxylate (412ml) was then added over 70min maintaining the temperature at <12°. The mixture was warmed to 21-25° and stirred for 1.5h. The reaction mixture was concentrated by distillation to a final volume of 2800ml. Methanol (2800ml) was added and the concentration repeated to a volume of 2800ml. More methanol (2000ml) was added and the mixture was heated to 55°. After 0.75h the slurry was cooled to 18° and then after a further 1h the product was collected. The bed was washed with methanol (2x1200ml) and then dried *in vacuo* at 40° to give the title compound (526.9g).

### Reference Example 5: Preparation of 2-{(2R)-3-[(3,4-dichlorobenzyl)(2-hydroxyethyl)amino]-2-hydroxypropyl}-1H-isoindole-1,3(2H)-dione

To a solution of 2-[(3,4-dichlorobenzyl)amino]ethanol (2.8g) in tetrahydrofuran (6.2 ml) is added (S)-2-(oxiran-2-ylmethyl)-1H-isoindole-1,3(2H)-dione (3.1 g) with stirring, under a nitrogen atmosphere. The mixture was heated to 90 °C over 1 h, then held at this temperature for 18 h. Further 2-[(3,4-dichlorobanzyl)amino]ethanol (0.14g) is added, and the reaction mixture heated to 90 °C for a further 5h. The reaction mixture is cooled to 22 °C, and diisopropyl ether (21 ml) added, and the product isolated by vacuum filtration. The filter cake is washed with diisopropyl ether (3 ml) and dried in vacuo at 40° to give the title compound as a white solid (4.79g).
LC/MS (System B) Rₜ 3.85mln, Mass Spectrum m/z 423 [MH⁺]

### Reference Example 6: 24(2R)-3[(3,4-dichlorobenzyl)(2-hydroxyethyl)amino]-2 hydroxypropyl}-1H-isoindole-1,3(2H)-dione (alternative method)

A suspension of of 2-[3,4-dichlorobenzyl)amino]ethanol (10.0g) and (*R*)*-*(-)-epichlorohydrin (3.6ml) in 1-propanol (2.5ml) was stirred at 15-25°C for 20h. The mixture was diluted with methyl tert-butyl ether (50ml) and treated with 2N hydrochloric acid (50ml). The layers were separated, and the organic phase was washed with further 2N hydrochloric acid (20ml). The combined aqueous phases were treated with 10N sodium hydroxide (17ml) and extracted with methyl tert-butyl ether (50ml). Further 10N sodium hydroxide (3ml) was added to the aqueous phase, and this was extracted with methyl tert-butyl ether (20ml). The combined organic phases were washed with water (40ml), and then concentrated to an oil. This was diluted with N,N-dimethylformamide (30ml) and treated with potassium phthalimide (8.3g). The mixture was heated for 3h at 110°C, cooled to 80°C and diluted with 1-propanol (30ml) and water (50ml). After cooling to 60°C, further water (20ml) was added, and the mixture was seeded with authentic 2-{(2*R*)-3-[(3,4-dichlorobenzyl)(2-hydroxyethyl)amino]-2-hydroxypropyl}-1*H*-isoindole-1,3(2*H*)-dione. The suspension was cooled to 0-5°C, isolated by filtration and washed with aqueous 1-propanol (2 x 30ml). The cake was dried in vacuo at 40° to give the title compound as a white solid (7.5g).

### Example 7: Preparation of 2-{[(2R)-4-(3,4-dichlorobenzyl)morpholin-2-yl]methyl}-1H-Isoindole-1,3(2H)-dione

A suspension of 2-{(2*R*)-3-[(3,4-dichlorobenzyl)(2-hydroxyethyl)amino]-2-hydroxypropy}-1*H*-Isoindole-1,3(2*H*)-dione (5.0g) and triphenylphosphine (3.57g) In tetrahydrofuran (25ml) was treated with a solution of diisopropylazodicarboxylate (2.67ml) in tetrahydrofuran (3ml) at 8-13°C over 18min. The reagent was rinsed in with tetrahydrofuran (2ml), and the mixture was warmed to 15-25°C and stirred for 1 hour. The reaction mixture was concentrated *in vacuo* to a paste, and resuspended in methanol (30ml) at 55-65°C. The suspension was stirred for 30min at this temperature, cooled to 5-10°C over 90min, and held at this temperature for 40min. The product was collected by filtration, washed with chilled methanol (15ml) and dried *in vacuo* at 40° to give the title compound as a white solid (4.08g).

### Description 1: Preparation of 4-({[({[(2S)-4-(3,4-dichlorobenzyl)morpholin-2-yl]methyl}amino)carbonyl]amino}methyl)benzamide benzenesulfonate hydrate

A solution of [(2S)-4-(3,4-dichlorobenzyl)morpholin-2-yl]methylamine (5g) In THF (10ml) is added to a slurry of N,N'-carbonyidiimidazole (3.2g) in THF (30ml) at 5-10 °C over ca. 10 min, The mixture is warmed to 15±3° and held at this temperature for ca. 15min. 4-Aminomethyl benzamide (3.0g) is then added, the mixture heated to 60±3° and stirred at this temp for 75 min.
The reaction is cooled to 22±3° and isopropyl acetate (40ml) added, followed by a solution of potassium dihydrogen phosphate (5% w/v. 40ml). The solution is filtered through celite (2g), the lower aqueous layer is removed and the upper organic layer washed with potassium dihydrogen phosphate (5% w/v, 2x40ml) then water (40ml). The organic phase is distilled at atmospheric pressure to remove THF and leave a slurry of 4-({[({[(2S)-4-(3,4-dichlorobenzyl)morpholin-2-yl]methyl}amino)carbonyl]amino}methyl)benzamide in isopropyl acetate (ca 60ml). This is cooled to 50±3° and isopropanol (30ml) is added, followed by an aqueous solution of benzene sulfonic acid (32% w/v, 10ml). The mixture is cooled to 22±3° over ca 1h, seeded with authentic 4-({[({[(2*S*)-4-(3,4-dichlorobenzyl)morpholin-2-yl]methyl] amino)carbonyl]amino}methyl)benzamide hydrate and aged at 22±3° for 72 h. The contents are cooled to 0±3° over 1 h and filtered. The filter cake is washed with a 4:1:0.1 mixture of isopropyl acetate/isopropyl alcohol/water (2.5ml) and dried in vacuo at 25±5° to give the title compound as a white solid (6.9g).
NMR (DMSO d-6): 2.81δ(1H) broad t; 3.0 - 3.4δ (5H) m; 3.67δ (2H) m; 4.02δ (1H) d of d, J=12.7Hz, 2,5Hz; 4.25δ (1H) d, 5.9Hz; 4.37δ (2H) m; 6.24δ (1 H) t. J=5.6Hz; 6,58δ (1H) t, J=5.9Hz; 7.3δ (6H) m; 7.48δ (1 H) d of d, J=8.3Hz, 2.0Hz; 7.61δ (2H) m [benzene sulphonate]; 7.75δ (1 H) d, J=8.3Hz; 7.81δ (1H) d, 2.0Hz; 7.82δ (2H) m; 7.91δ (1 H) broad s; 9.85 (1 H) broad s [NH⁺].

### Description 2: Preparation of N-{[(2S)-4-(3,4-dichlorobenzyl)-2-morpholinyl]methyl}-N'-[(2-methyl-2H-tetrazol-5yl)methyl]

A suspension of 1-[(2S)-4-(3,4-dichlorobenzyl)morpholin-2-yl]methylamine, 1:1 salt with D-tartaric acid (70g) in water (350ml) and dichloromethane (420ml) was treated with 35% aqueous ammonia (35ml) at <10°C. The aqueous phase was washed with further dichloromethane (70ml), and the combined organic phases were washed with water (70ml). The organic solution was concentrated to low volume, diluted with tetrahydrofuran (420ml) and reconcentrated to low volume. This solution in tetrahydrofuran was added to a suspension of carbonyl diimidazole (29.8g) in tetrahydrofuran (455ml) over ca 20min at 0 - 5°C. The mixture was warmed to 10-15°C, and held for 30min. Isopropanol (21 ml) was added over 5min, and the mixture was stirred for a further 20min at 10-15°C. 1-(2-Methyl-2H-tetrazol-5yl)methanamine hydrochloride (25.2g) was added, and the mixture was heated to 55-60°C, and held for 5h. Tetrahydrofuran (200ml) was removed by distillation, and the mixture was cooled to 40-45°C. The mixture was treated with 12% aqueous potassium dihydrogen phosphate (360ml), and the aqueous phase was removed. The organic phase was diluted with ethyl acetate (175ml) and washed with 3%w/w aqueous sodium chloride (175ml), The organic phase was warmed to 40-45°C, filtered and washed with tetrahydrofuran (70ml). The solution was concentrated to low volume by distillation, diluted with isopropanol (595ml), and concentrated to low volume by distillation. Further isopropanol (770ml) was added, the mixture was heated to 75°C and cooled to 60-65°C. The solution was seeded, heated at 30-60°C for 18h, and then cooled to 10-15°C. The product was isolated by filtration, washed with isopropanol and dried in vacuo to give the title compound as a white solid (54.4g).
LC-MS (System A) Rt 2.21min, mass spectrum 414/416, MH+

### Description 3: N-{[(2S)-4-(3,4-Dichlorobenzyl)morpolin-2-yl]methyl}-2-(5-phenyl-2H-tetraazol-2-yl)acetamide

A solution of (5-phenyl-2H-tetraazol-2-yl)acetic acid (0.082g) In N,N-dimethylformamide (2ml) under nitrogen was treated with O-(7-azabenzotriazol-1-yl)-N,N,N'N'-tetramethylammonium hexafluorophosphate (0.152g) and N,N-diisopropylethylamine (0.139ml) followed by a solution of 1-[(2S)-4-(3,4-dichlorobenzyl)morpholin-2-yl]methanamine (0.110g) in N,N-dimethylformamide (3ml), and the mixture was stirred at 22°C for 4h. The solvent was removed in vacuo and the residue dissolved in ethyl acetate (20ml). The solution was washed with 10% aqueous citric acid (20ml), brine (20ml), saturated aqueous sodium hydrogen carbonate (20ml) and brine (20ml), dried (MgSO₄) and evaporated in vacuo. Purification by flash chromatography on silica gel (Merck 9385), eluting with ethyl acetate, followed by trituration of the resultant product with diethyl ether, gave the title compound as a white solid (0.184g).
LC-MS (System A): Rt 2.85min. Mass Spectrum *m*/*z* 461 [MH⁺].

## Claims

1. A process for the preparation of a compound of formula (IIIA) 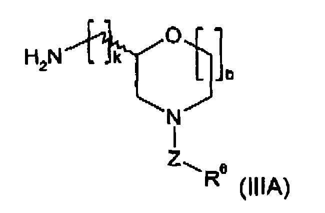 or a salt thereof; wherein;
Z represents a bond, CO, SO₂, CR¹⁰R⁷(CH₂)ₙ, (CH₂)ₙCR¹⁰R⁷, CHR⁷(CH₂)ₙO, CHR⁷(CH₂)ₙS, CHR⁷(CH₂)ₙOCO, CHR⁷(CH₂)ₙCO, COCHR⁷(CH₂)ₙ or SO₂CHR⁷(CH₂)ₙ;
R⁶ represents C₁₋₆ alkyl, C₂₋₆ alkenyl, aryl, heteroaryl, aryl-C₂₋₆ alkenyl-, -CN or a group of formula -Y²-J³;
R⁷ represents hydrogen, C₁₋₄ alkyl, CONR⁸R⁹ or COOC₁₋₆ alkyl;
a and b represent 1 or 2, such that a+b represents 2 or 3;
n represents an integer from 0 to 4;
J³ represents a moiety of formula (K); 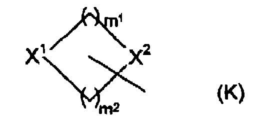 wherein X¹ represents oxygen, NR¹¹ or sulphur, X² represents CH₂, oxygen, NR¹² or sulphur, m¹ represents an integer from 1 to 3 and m² represents an integer from 1 to 3, provided that m¹+m² is in the range from 3 to 5, also provided that when both X¹ and X² represent oxygen, NR¹¹, NR¹² or sulphur, m¹ and m² must both not equal less than 2, wherein K is optionally substituted by one or more (eg. 1 or 2) -Y³-aryl, -Y³-heteroaryl, -Y³-CO-aryl, -COC₃₋₈ cycloalkyl, -Y³-CO-heteroaryl, -C₁₋₈ alkyl, -Y³-COOC₁₋₆ alkyl, -Y³-COC₁₋₆ alkyl, -Y³-W, -Y³-CO-W, -Y³-NR¹⁵R¹⁶, -Y³-CONR¹⁵R¹⁶, hydroxy, oxo, -Y³-SO₂NR¹⁵R¹⁶, -Y³-SO₂C₁₋₆ alkyl, -Y³-SO₂aryl, -Y³-SO₂heteroaryl, -Y³-NR¹³C₁₋₆ alkyl, -Y³-NR¹³SO₂C₁₋₆ alkyl, -Y³-NR¹³CONR¹⁵R¹⁶, -Y³-NR¹³COOR¹⁴ or -Y³-OCONR¹⁶R¹⁶ groups, and is optionally fused to a monocyclic aryl or heteroaryl ring;
R⁸, R⁹, R¹⁰, R¹¹, R¹², R¹³ and R¹⁴ independently represent hydrogen or C₁₋₆ alkyl;
R¹⁵ and R¹⁶ independently represent hydrogen or C₁₋₆ alkyl or R¹⁵ and R¹⁶ together with the nitrogen atom to which they are attached may form a morpholine, piperidine or pyrrolidine ring;
R¹⁷ and R¹⁸ independently represent hydrogen or C₁₋₆ alkyl;
W represents a saturated or unsaturated, non-aromatic 5-7 membered ring containing between 1 and 3 heteroatoms selected from nitrogen, oxygen or sulphur, optionally substituted with one or more C₁₋₆ alkyl, halogen or hydroxy groups;
Y¹, Y² and Y³ independently represent a bond or a group of formula - (CH₂)ₚCR^{c}R^{d}(CH₂)_{q}- wherein R^{c} and R^{d} independently represent hydrogen or C₁₋₄ alkyl or R^{c} and R^{d} may together with the carbon atom to which they are attached form a C₃₋₅ cycloalkyl group, and p and q independently represent an integer from 0 to 5 wherein p + q is an integer from 0 to 5;, and;
k is 1 or 2;
which process comprises the reaction of a compound of formula (XX) 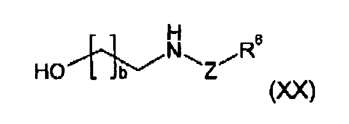 wherein,
b, Z, and R⁶ are as defined for formula (IIIA);
with an enantiomer of a compound of formula (XXI), under Mitsonobu conditions, 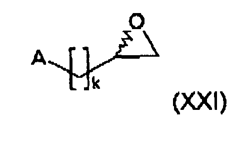 wherein;
A is a protected amino group and k is 1 or 2;
followed by deprotection of the amino group to give a compound of formula (IIIA),

2. A process according to claim 1 wherein b represents 1,

3. A process according to claim 1 or claim 2 wherein Z represents CH₂.

4. A process according to any one of claims 1 to 3 wherein R⁶ represents phenyl substituted by one or more chlorine or fluorine groups.

5. A process according to claim 4 wherein R⁶ represents dichlorophenyl, 3-chlorophenyl, 5-chlorothiophenyl, 4-fluorophenyl or 3,4-difluorophenyl.

6. A process according to claim 5 wherein R⁶ represents 3,4-dichlorophenyl.

7. A process according to claim 1 wherein b represents 1, Z represents -CH₂-, R⁶ represents 3,4-dichlorophenyl, and k represents 1.

## Patentansprüche

1. Verfahren zur Herstellung einer Verbindung der Formel (IIIA): 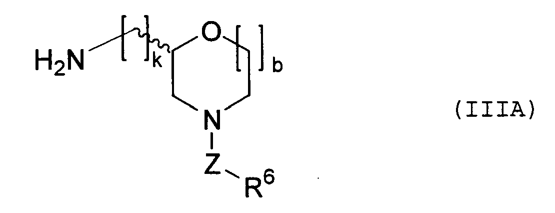 oder eines Salzes davon,
worin
Z eine Bindung, CO, SO₂, CR¹⁰R⁷(CH₂)ₙ, (CH₂)ₙCR¹⁰R⁷, CHR⁷(CH₂)ₙO, CHR⁷(CH₂)ₙS, CHR⁷(CH₂)ₙOCO, CHR⁷(CH₂)ₙCO, COCHR⁷(CH₂)ₙ oder SO₂CHR⁷(CH₂)ₙ darstellt;
R⁶ C₁₋₆-Alkyl, C₂₋₆-Alkenyl, Aryl, Heteroaryl, Aryl-C₂₋₆-alkenyl-, -CN oder eine Gruppe der Formel -Y²-J³ darstellt;
R⁷ Wasserstoff, C₁₋₄-Alkyl, CONR⁸R⁹ oder COOC₁₋₆-alkyl darstellt;
a und b 1 oder 2 darstellen, so daß a+b 2 oder 3 darstellt;
n eine ganze Zahl von 0 bis 4 darstellt;
J³ einen Rest der Formel (K) darstellt: 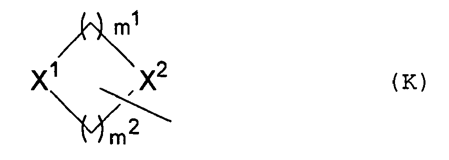 worin X¹ Sauerstoff, NR¹¹ oder Schwefel darstellt, X² CH₂, Sauerstoff, NR¹² oder Schwefel darstellt, m¹ eine ganze Zahl von 1 bis 3 darstellt und m² eine ganze Zahl von 1 bis 3 darstellt, vorausgesetzt, daß m¹+m² im Bereich von 3 bis 5 ist, ebenfalls vorausgesetzt, daß, wenn beide X¹ und X² Sauerstoff, NR¹¹, NR¹² oder Schwefel darstellen, dann müssen m¹ und m² beide nicht weniger als 2 gleichen, worin K gegebenenfalls mit einem oder mehreren (z.B. 1 oder 2) -Y¹-Aryl-, -Y³-Heteroaryl-, -Y³-CO-Aryl-, -COC₃₋₆-Cycloalkyl-, -Y³-CO-Heteroaryl-, -C₁₋₆-Alkyl-, -Y³-COOC₁₋₆-Alkyl-, -Y³-COC₁₋₆-Alkyl-, -Y³-W-, -Y³-CO-W-, -Y³-NR¹⁵R¹⁶-, -Y³-CONR¹⁵R¹⁶-, Hydroxy-, Oxo-, -Y³-SO²NR¹⁵R¹⁶-, -Y³-SO₂C₁₋₆-Alkyl-, -Y³-SO₂aryl-, -Y³-SO₂heteroaryl-, -Y³-NR¹³C₁₋₆-Alkyl-, -Y³-NR¹³SO₂C₁₋₆-Alkyl-, -Y³-NR¹³CONR¹⁵R¹⁶-, -Y³-NR¹³COOR¹⁴- oder -Y³-OCONR¹⁶R¹⁶-Gruppen substituiert ist, und gegebenenfalls an einen monocyclischen Aryl- oder Heteroarylring kondensiert ist;
R⁸, R⁹, R¹⁰, R¹¹, R¹², R¹³ und R¹⁴ unabhängig Wasserstoff oder C₁₋₆-Alkyl darstellen;
R¹⁵ und R¹⁶ unabhängig Wasserstoff oder C₁₋₆-Alkyl darstellen, oder R¹⁵ und R¹⁶ zusammen mit dem Stickstoffatom, an das sie gebunden sind, einen Morpholin-, Piperidin- oder Pyrrolidinring bilden können;
R¹⁷ und R¹⁸ unabhängig Wasserstoff oder C₁₋₆-Alkyl darstellen;
W einen gesättigten oder ungesättigten nichtaromatischen Ring mit 5-7 Gliedern darstellt, der zwischen 1 und 3 Heteroatome enthält, die aus Stickstoff, Sauerstoff oder Schwefel ausgewählt sind, gegebenenfalls mit einem oder mehreren C₁₋₆-Alkyl-, Halogen- oder Hydroxygruppen substituiert;
Y¹, Y² und Y³ unabhängig eine Bindung oder Gruppe der Formel -(CH₂)ₚCR^{c}R^{d}(CH₂)_{q}- darstellen, worin R^{c} und R^{d} unabhängig Wasserstoff oder C₁₋₄-Alkyl darstellen, oder R^{c} und R^{d} zusammen mit dem Kohlenstoffatom, an das sie gebunden sind, eine C₃₋₆-Cycloalkylgruppe bilden können, und p und q unabhängig eine ganze Zahl von 0 bis 5 darstellen, worin p+q eine ganze Zahl von 0 bis 5 ist; und
k 1 oder 2 ist;
wobei das Verfahren das Umsetzen einer Verbindung der Formel (XX): 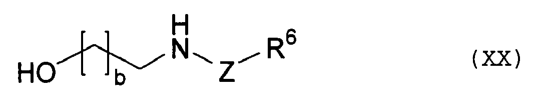 worin
b, Z und R⁶ wie für Formel (IIIA) definiert sind;
mit einem Enantiomer einer Verbindung der Formel (XXI) unter Mitsonobu-Bedingungen: 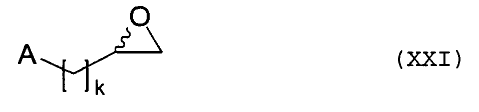 worin
A eine geschützte Aminogruppe ist und k 1 oder 2 ist;
gefolgt von Entschützen der Aminogruppe umfaßt, um eine Verbindung der Formel (IIIA) zu ergeben.

2. Verfahren gemäß Anspruch 1, worin b 1 darstellt.

3. Verfahren gemäß Anspruch 1 oder Anspruch 2, worin Z CH₂ darstellt.

4. Verfahren gemäß einem der Ansprüche 1 bis 3, worin R⁶ Phenyl darstellt, das mit einer oder mehreren Chlor- oder Fluorgruppen substituiert ist.

5. Verfahren gemäß Anspruch 4, worin R⁶ Dichlorphenyl, 3-Chlorphenyl, 5-Chlorthiophenyl, 4-Fluorphenyl oder 3,4-Difluorphenyl darstellt.

6. Verfahren gemäß Anspruch 5, worin R⁶ 3,4-Dichlorphenyl darstellt.

7. Verfahren gemäß Anspruch 1, worin b 1 darstellt, Z -CH₂-darstellt, R⁶ 3,4-Dichlorphenyl darstellt und k 1 darstellt.

## Revendications

1. Procédé de préparation d'un composé de formule (IIIA) 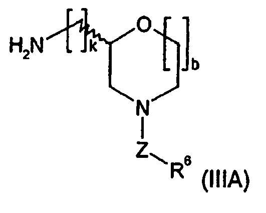 ou d'un sel de celui-ci ;
dans laquelle :
Z représente une liaison, CO, SO₂, CR¹⁰R⁷(CH₂)ₙ, (CH₂)ₙCR¹⁰R⁷, CHR⁷(CH₂)ₙO, CHR⁷(CH₂)ₙS, CHR⁷ (CH2)ₙOCO, CHR⁷(CH₂)ₙCO, COCHR⁷(CH₂)ₙ ou SO₂CHR⁷ (CH₂)ₙ;
R⁶ représente un groupe alkyle en C₁ à C₆, alcényle en C₂ à C₆, aryle, hétéroaryle, arylalcényl en C₂ à C₆, -CN ou un groupe de formule -Y²-J³ ;
R⁷ représente un atome d'hydrogène, un groupe alkyle en C₁ à C₄, CONR⁸R⁹ ou COO-alkyle en C₁ à C₆ ;
a et b représentent 1 ou 2, de telle manière que a + b représente 2 ou 3 ;
n représente un nombre entier de 0 à 4 ;
J³ représente un radical de formule (K) : 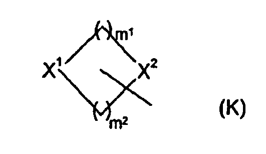 dans laquelle X¹ représente un atome d'oxygène, NR¹¹ ou un atome de soufre, X² représente CH₂, un atome d'oxygène, NR¹² ou un atome de soufre, m¹ représente un nombre entier de 1 à 3 et m² représente un nombre entier de 1 à 3, à condition que m¹ + m² se situe dans la plage de 3 à 5, également à condition que lorsque tous les deux de X¹ et X² représentent un atome d'oxygène, NR¹¹, NR¹² ou un atome de soufre, m¹ et m² ne doivent pas être égaux à moins de 2, où K est éventuellement substitué par un ou plusieurs (par exemple, 1 ou 2) groupes -Y³-aryle, -Y³-hétéroaryle, -Y³-CO-aryle, -CO-cycloalkyle en C₃ à C₆, -Y³-CO-hétéroaryle, alkyle en C₁ à C₆, -Y³-COO-alkyle en C₁ à C₆, -Y³-CO-alkyle en C₁ à C₆, -Y³-W, -Y³-CO-W, -Y³-NR¹⁵NR¹⁶, -Y³-CONR¹⁵R¹⁶, hydroxy, oxo, -Y³-SO₂NR¹⁵R¹⁶, -Y³-SO₂-alkyle en C₁ à C₆, -Y³-SO₂-aryle, -Y³-SO₂-hétéroaryle, -Y³-NR¹³-alkyle en C₁ à C₆, -Y³-NR¹³SO₂-alkyle en C₁ à C₆, -Y³-NR¹³CONR¹⁵R¹⁶, -Y³-NR¹³COOR¹⁴ ou -Y³-OCONR¹⁵R¹⁶, et est éventuellement fusionné à un cycle aryle ou hétéroaryle monocyclique ;
R⁸, R⁹, R¹⁰, R¹¹, R¹², R¹³ et R¹⁴ représentent indépendamment un atome d'hydrogène ou un groupe alkyle en C₁ à C₆ ;
R¹⁵ et R¹⁶ représentent indépendamment un atome d'hydrogène ou un groupe alkyle en C₁ à C₆ ou R¹⁵ et R¹⁶ conjointement avec l'atome d'azote auquel ils sont fixés peuvent former un cycle morpholine, pipéridine ou pyrrolidine ;
R¹⁷ et R¹⁸ représentent indépendamment un atome d'hydrogène ou un groupe alkyle en C₁ à C₆ ;
W représente un cycle de 5 à 7 chaînons non aromatique, saturé ou insaturé, contenant entre 1 et 3 hétéroatomes choisis parmi l'azote, l'oxygène ou le soufre, éventuellement substitué par un ou plusieurs groupes alkyle en C₁ à C₆, halogène ou hydroxy ;
Y¹, Y² et Y³ représentent indépendamment une liaison ou un groupe de formule -(CH₂)ₚCR^{c}R^{d}(CH₂)_{q}- où R^{c} et R^{d} représentent indépendamment un atome d'hydrogène ou un groupe alkyle en C₁ à C₄ ou R^{c} et R^{d} peuvent conjointement avec l'atome de carbone auquel ils sont fixés former un groupe cycloalkyle en C₃ à C₆, et p et q représentent indépendamment un nombre entier de 0 à 5 où p + q est un nombre entier de 0 à 5 ; et ;
k vaut 1 ou 2 ;
lequel procédé comprend la réaction d'un composé de formule (XX) 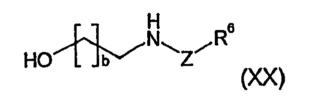 dans laquelle :
b, Z et R⁶ sont tels que définis pour la formule (IIIA) ;
avec un énantiomère d'un composé de formule (XXI), dans des conditions de Mitsonobu, 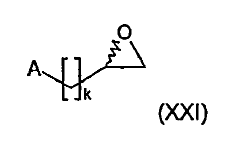
dans laquelle :
A représente un groupe amino protégé et k vaut 1 ou 2 ;
ceci suivi d'une déprotection du groupe amino pour donner un composé de formule (IIIA).

2. Procédé selon la revendication 1, dans lequel b représente 1.

3. Procédé selon la revendication 1 ou la revendication 2, dans lequel Z représente CH₂.

4. Procédé selon l'une quelconque des revendications 1 à 3, dans lequel R⁶ représente un groupe phényle substitué par un ou plusieurs groupes de chlore ou de fluor.

5. Procédé selon la revendication 4, dans lequel R⁶ représente un groupe dichlorophényle, 3-chlorophényle, 5-chlorothiophényle, 4-fluorophényle ou 3,4-difluorophényle.

6. Procédé selon la revendication 5, dans lequel R⁶ représente un groupe 3,4-dichlorophényle.

7. Procédé selon la revendication 1, dans lequel b représente 1, Z représente -CH₂-, R⁶ représente un groupe 3,4-dichlorophényle et k représente 1.
